# EUROPÄISCHE PATENTSCHRIFT

(11) **EP 3 954 299 B1**
(45) Veröffentlichungstag und Bekanntmachung des Hinweises auf die Patenterteilung: **06.12.2023**
(21) Anmeldenummer: 20190939.7
(22) Anmeldetag: 13.08.2020
(51) Int. Cl.: A61B 8/04, A61B 8/06, A61B 8/08, A61B 5/02

(54) **VERFAHREN ZUR BESTIMMUNG DER LASTUNABHÄNGIGEN KONTRAKTILITÄT**
METHOD FOR DETERMINING LOAD-INDEPENDENT CONTRACTILITY
PROCÉDÉ DE DÉTERMINATION DE LA CONTRACTILITÉ À CHARGE INDÉPENDANTE

(43) Veröffentlichungstag der Anmeldung: 16.02.2022
(73) Patentinhaber: Justus-Liebig-Universität Gießen, 35390 Gießen (DE)
(72) Erfinder: Tello, Khodr, 35423 Lich (DE); Gall, Henning, 35452 Heuchelheim (DE); Ghofrani, Ardeschir, 35435 Wettenberg (DE); Seeger, Werner, 35444 Biebertal (DE)
(74) Vertreter: Stumpf, Peter

(56) Entgegenhaltungen:
- DE-A1-102013 004 110
- ETIENNE GAYAT ET AL: "Noninvasive quantification of left ventricular elastance and ventricular-arterial coupling using three-dimensional echocardiography and arterial tonometry", AMERICAN JOURNAL OF PHYSIOLOGY HEART AND CIRCULATORY PHYSIOLOGY, Bd. 301, Nr. 5, 1. November 2011 (2011-11-01), Seiten H1916-H1923, XP055764035, US ISSN: 0363-6135, DOI: 10.1152/ajpheart.00760.2011
- Chen-Huan Chen ET AL: "New Methods Noninvasive Single-Beat Determination of Left Ventricular End-Systolic Elastance in Humans", , 1. Dezember 2001 (2001-12-01), XP055763940, Gefunden im Internet: URL:https://core.ac.uk/download/pdf/823324 17.pdf [gefunden am 2021-01-12]

## Beschreibung

Die Erfindung betrifft ein Verfahren zur Bestimmung der lastunabhängigen Kontraktilität (endsystolic elastance Ees) einer Herzkammer. Die Bestimmung erfolgt nur auf Grundlage von Ultraschallmessungen.

### Beschreibung und Einleitung des allgemeinen Gebietes der Erfindung

Lastunabhängige Parameter der Herzstärke der rechten Herzkammer sind von wichtiger Bedeutung zur Charakterisierung von Patienten mit Lungenerkrankungen, insbesondere Patienten mit Lungenhochdruck. Die Rechtsherzfunktion ist der größte Prognosefaktor dieser Patienten.

Die Ultraschallkardiographie (UKG), auch Herzultraschall genannt, unterstützt Ärzte bei der genauen Diagnose vorliegender Herzprobleme.

Ultraschall (Sonographie) funktioniert mittels Schallwellen. Die Frequenz liegt dabei bei von 1 bis 3 MHz. Diese Ultraschallwellen werden vom Schallkopf des Ultraschallgeräts ausgesendet und wieder aufgenommen. Verschiedene Gewebe reflektieren den Schall unterschiedlich gut. Aus den Informationen kann ein Bild von inneren Bereichen des Körpers in Echtzeit erstellt werden.

Eine Ultraschallkardiographie bietet einige Vorzüge:
Die Untersuchung ist strahlungsfrei. Es gibt keine Nebenwirkungen durch einen invasiven Eingriff. Weiterhin eignet sich die Ultraschalluntersuchung auch für Schwangere und Patienten mit einer Kontrastmittel-Allergie. Der Patient kann sich während der Behandlung bewegen (im Vergleich zu einer Computer- oder Kernspintomografie).

Es gibt zwei Möglichkeiten einen Herzultraschall durchzuführen: die meistens eingesetzte transthorakale Echokardiographie und die seltener gebrauchte transösophageale Echokardiographie.

### Transthorakale Echokardiographie (TTE) - Herzecho von außen

Zu den Standardvarianten, um Herzprobleme festzustellen, gehört die transthorakale Echokardiographie (TTE). Dabei legt der Facharzt den Ultraschallkopf auf die vordere Brustwand und kann so anhand des Ultraschallbildes oder Kardiogramm Unregelmäßigkeiten in den Koronargefäßen erkennen. Die Methode ist vergleichbar mit einem herkömmlichen Ultraschall, wobei es verschiedene Techniken gibt, mit denen man genaue Untersuchungsergebnisse erzielt. Dazu zählen:

### (Farb-)Doppler-Echokardiographie:

Die Doppler-Echokardiografie oder auch Farb-Doppler-Echokardiographie bestimmt die Blutgeschwindigkeit. Durch das Messen der Strömungsgeschwindigkeit und das Nachweisen der Strömungsbeschleunigungen wird die Funktion der Herzklappen kontrolliert. Bei der farbkodierten Echokardiographie wird im Kardiogramm mit roter und blauer Farbe die Blutflussrichtung besser erkennbar gemacht: Das Fließen des Blutes hin zum Schallkopf wird als rote Wolke verbildlicht und weg vom Schallkopf als blaue Wolke. Durch dieses Verfahren ist der Arzt in der Lage, abzuschätzen, ob und wie undicht die Herzklappen sind.

### 3D-Abbildung des Herzens:

Wenn Patienten an einer Herzschwäche leiden oder die Herzklappen genauer betrachtet werden sollen, dann empfiehlt sich eine spezielle Sonde, die dreidimensionale, also räumliche Darstellungen ermöglicht. Sie vereinfacht die Beurteilung der Herzfunktion in Echtzeit. 3D-Messungen sind durch ihre Matrix-Schallkopftechnologie um einiges genauer als 2D-Aufnahmen, insbesondere da diese Untersuchungstechnik auch in der Lage ist, Volumen der Kammern und Vorhöfe zu messen.

### Stressechokardiographie/Belastungsechokardiographie:

Eine Stressechokardiographie, oft auch als Stress-Echo bezeichnet, ähnelt einem Belastungs-EKG, erfolgt aber mit Ultraschall. Der Körper des Patienten wird durch gefäßerweiternde Medikamente oder körperliche Anstrengung belastet. Häufiger findet die Fahrrad-Stressechokardiographie Anwendung: Der Patient liegt auf einer besonderen, kippbaren Echo-Liege und muss in die Pedale treten. Dabei wird die Belastung stufenweise gesteigert. Einsatz von Kontrastmittel: Ab und an verwendet man Ultraschall-Kontrastmittel bei Echokardiographien. So kann unter anderem die Durchblutung der Blutgefäße besser dargestellt und untersucht werden. Das hierbei verwendete Kontrastmittel ist für Patienten verträglicher als das bei Röntgen oder MRTs verwendete.

### Transösophageale Echokardiographie (TEE) - Schluckecho:

Die Methode der transösophagealen Echokardiographie (TEE - von der englischen Bezeichnung "transesophageal echocardiography"; im Deutschen auch TOE) findet seltener Anwendung. Sie wird umgangssprachlich auch Schluckecho genannt, da ein flexibler Schlauch - ähnlich wie bei einer Magenspiegelung - über den Mund in die Speiseröhre eingeführt wird. Um einen eventuell aufkommenden Würgereiz zu vermeiden, sprüht der Arzt ein lokales Betäubungsmittel in den Rachen.

An der Spitze des Schlauchs befindet sich eine Ultraschallsonde. Der Vorteil: Die Nähe der Speiseröhre zum Herzen ermöglicht deutlichere Aufnahmen (zum Beispiel des linken Vorhofs oder etwaiger Blutgerinnselbildungen bei Herzrhythmusstörungen). Auch angeborene Herzfehler erkennt der Arzt durch die transösophageale Echokardiographie leichter.

Die sogenannte ventrikuloarterielle Kopplung repräsentiert das Zusammenspiel aus Herzkammer (Ventrikel) und nachgeschalteter Arterie und dient u.a. zur Beurteilung der Kontraktilität bzw. der Herzstärke im Vergleich zu der Nachlast in der Arterie. Dieses Maß wird sowohl in der linken als auch in der rechten Herzkammer gemessen.

Bei der ventrikulär-peripheren arteriellen Kopplung wird das Kreislaufsystem als Ganzes betrachtet. Ventrikel und Arterie können als ein elastisches System betrachtet werden, ihre Wirkungen beeinflussen sich gegenseitig. Die diastolische und systolische Funktion des Ventrikels kann innere Organe, einschließlich des Ventrikels selbst und der distalen Gliedmaßen, mit Blut versorgen. Die Regeln der ventrikulären Kontraktion und der peripheren arteriellen Kopplung können bestimmt werden, wenn das ventrikuläre Volumen und die Volumen- und/oder Druckinformationen der peripheren Arterien gleichzeitig erhalten werden. Unter den zahlreichen kardialen Funktionstests werden Schlagvolumen und Auswurffraktion (Ejektionsfraktion) usw. von der kardialen Vorlast, Nachlast und Herzfrequenz beeinflusst. Diese Funktionstests können daher nicht dazu verwendet werden, die spezifische myokardiale Kontraktilität zu zeigen. Zahlreiche Daten zeigen, dass die Beziehung zwischen endsystolischem Druck und Volumen eine bedeutende Rolle bei der Beurteilung des Status der systolischen Funktion spielt. Die durch spezielle Herz-Volumen-Katheter (sog Conductance-Katheter) gemessene maximale Elastizität (Emax) ist eine Steigung der ventrikulären end-systolischen Druck-Volumen-Relationslinie, die ein spezifischer Index ist, der die ventrikuläre Kontraktilität zeigt. Aufgrund der Invasivität und des teuren Preises ist diese Herzkatheterisierung im klinischen Umfeld jedoch schwierig durchführbar.

Dementsprechend ist es notwendig, ein medizinisches Ultraschallüberwachungsgerät und -verfahren zur Verfügung zu stellen, mit dem myokardmechanische Parameter auf weniger invasive Weise erfasst werden können.

### Stand der Technik

Die lastunabhängige Kontraktilität kann insbesondere für die rechte Herzkammer noch nicht mittels Ultraschalluntersuchungsmethoden bestimmt werden.

Die lastunabhängige Kontraktilität wird derzeitig mittels Druck-Volumen-Katheter bestimmt. Beispiel sei hierzu auf die Schrift: Funktionsanalyse der rechten Herzkammer bei Kindern mit angeborenen Herzfehlern mithilfe der Conductance-Technik Journal für Kardiologie - (Apitz C et al. Austrian Journal of Cardiology 2009; 16 (7-8): 264-269) verwiesen.

Diese Methode ist klinisch erprobt und zuverlässig einsetzbar und stellt auch den Goldstandard dar. Sie weist jedoch in ihrer Anwendung auch Nachteile auf. So sind die Kosten eines einzelnen Katheters erheblich. Weiterhin wird dieser Katheter invasiv eingesetzt und dies ist für einen Patienten sehr belastend.

Weiterhin offenbart die Schrift NE GAYAT ET AL: "Noninvasive quantification of left ventricular elastance and ventricular-arterial coupling using three-dimensional echocardiography and arterial tonometry",AMERICAN JOURNAL OF PHYSIOLOGY HEART AND CIRCULA-TORY PHYSIOLOGY,Bd. 301, Nr.5, 1. November 2011 (2011 -11 -01 ), Seiten H1916-H1923,XP055764035,us ISSN: 0363-6135, DOI: 10.1152/ajpheart.00760.2011eine übliche Ultraschallüberwachungsvorrichtung umfassend
a. ein Ultraschallmesselement welches so an einer Körperoberfläche eines zu untersuchenden Patienten anbringbar oder in den Patienten einbringbar und so ausgebildet ist, dass es ein Echosignal erfassen kann,
b. ein Blutdruckmesselement, welches so ausgebildet ist, dass es wenigstens einen Blutdruckparameter eines zu untersuchenden Patienten messen kann,
c. ein Verarbeitungselement, welches so ausgebildet, dass es ein Echosignal des Ultraschallmesselements empfangen und das Echosignal in wenigstens einen Blutflussparameter umwandeln kann und ausgebildet ist, dass es wenigstens einen myokardmechanischen Parameter aus wenigstens einem Blutflussparameter und wenigstens einem Blutdruckparameter berechnen kann,
d. ein Anzeigeelement, welches so ausgebildet ist, dass es den Blutdruck, wenigstens einen Blutflussparameter und wenigstens einen myokardmechanischen Parameter des Verarbeitungselement empfangen und anzeigen kann.

### Aufgabe

Aufgabe der vorliegenden Erfindung ist es, die Nachteile des Standes der Technik zu vermeiden, sodass Ultraschalluntersuchungsmethoden zur Bestimmung der lastunabhängigen Kontraktilität Ees genutzt werden können. Damit wird eine präzise und zuverlässige Diagnose ermöglicht, die nichtinvasiv am Patienten durchführbar ist.

### Lösung der Aufgabe

Die Lösung dieser Aufgaben ergibt sich aus den Merkmalen des Anspruchs.

Dieses Verfahren ist sowohl für die rechte wie auch für die linke Herzkammer durchführbar. Insbesondere für die rechte Herzkammer ist dieses Verfahren höchst interessant, da eine komplette nicht-invasive Kontraktilitätsmessung möglich ist. Die Messung kann dabei vollständig mittels Ultraschalles erfolgen. An der Schwierigkeit der Messung im Goldstandard ist bislang die Erfassung der Kontraktilität gescheitert. Wegen seiner komplexen Geometrie galt die Funktionsanalyse des rechten Ventrikels schon immer als eine Herausforderung. Während die Form der linken Herzkammer einem Ellipsoid gleicht, hat der rechte Ventrikel aus seitlicher Betrachtung eine mondsichelähnliche Form und von anterior erscheint er annähernd dreieckig zu sein. Dadurch lassen sich bisher für die linke Herzkammer verwendeten Messverfahren nicht ohne weiteres auf die rechte Herzkammer übertragen.

Als Maß für die Kontraktilität eines Ventrikels wird im Rahmen des erfindungsgemäßen Verfahrens die endsystolische Druck-Volumen-Beziehung (Elastance) verwendet. Untersuchungen am linken Ventrikel haben gezeigt, dass eine enge Beziehung zwischen der end-systolischen Druck-Volumen-Beziehung und der Kraft-Länge-Beziehung der isolierten Herzmuskelzelle bzw. einer einzelnen Papillarmuskelfaser besteht. Weil die maximale Kraft, die durch eine Herzmuskelzelle mit einer vorgegebenen Länge entwickelt wird, repräsentativ für ihre kontraktile Funktion ist, ist es allgemein akzeptiert, dass die endsystolische Druck-Volumen-Beziehung die zuverlässigste Darstellung der mechanischen kontraktilen Leistungsfähigkeit des Myokards darstellt, die man in vivo, also im intakten Kreislauf, erhalten kann.

Zunächst werden einige zum Verständnis des erfindungsgemäßen Verfahrens notwenige Begrifflichkeiten kurz erläutert.

Isovolumetrischen Kontraktion (IVK): Die Ventrikel kontrahieren sich, wodurch der Innendruck steigt. Dies führt zum sofortigen Verschluss der Segelklappen bzw. Atrioventrikular-(AV-)Klappen, also der Mitral- und der Trikuspidalklappe. Die Klappen schließen sich, da der Ventrikeldruck den Vorhofdruck übersteigt. Damit wird der Rückfluss von Blut in den Vorhof verhindert. Zu diesem Zeitpunkt sind alle 4 Herzklappen geschlossen - d.h. der Druck in den Ventrikeln steigt an, während das Volumen gleichbleibt. Diese Phase ist die isovolumetrischen Kontraktion.

Isovolumetrische Relaxation (IVR): Wenn die Kontraktion der Ventrikel nachlässt und der Innendruck unter den Aortendruck absinkt, schließen sich die Taschenklappen wieder. Da zu diesem Zeitpunkt alle 4 Herzklappen geschlossen sind und sich somit das Volumen im Ventrikel nicht verändert, bezeichnet man diese Phase - analog zur Anspannungsphase - als isovolumetrischen. Während der Entspannungsphase fällt der Ventrikeldruck rasch ab. Diese Phase wird auch als isovolumetrische Relaxation bezeichnet.

Regurgitationsfluss: Als Regurgitationsfluss bezeichnet man den durch eine Herzklappe zurückfließenden Blutstrom.

Endsystolischer Druck (Pes): Der endsystolische Druck ist der Druck, der am Ende der Anspannungsphase des Herzens (Systole) in einem der beiden Herzventrikel herrscht. Pes ist der Druck-Punkt, an dem das Volumen den niedrigsten Wert hat. Der maximale rechtsventrikuläre Druck Pmax ist der maximal mögliche endsystolische Druck Pes im rechten Ventrikel.

Endsystolisches Volumen (Ves): Das endsystolische Volumen ist das Blutvolumen, das am Ende der Systole nach maximaler Entleerung eines Ventrikels, d.h. nach der vollständigen Kammerkontraktion, in einer Herzkammer vorhanden ist.
systolischer pulmonalarterieller Druck (sPAP): Der systolische pulmonalarterielle Druck ist der Blutdruck in der Lungenarterie während das Herz sich zusammenzieht (Systole).

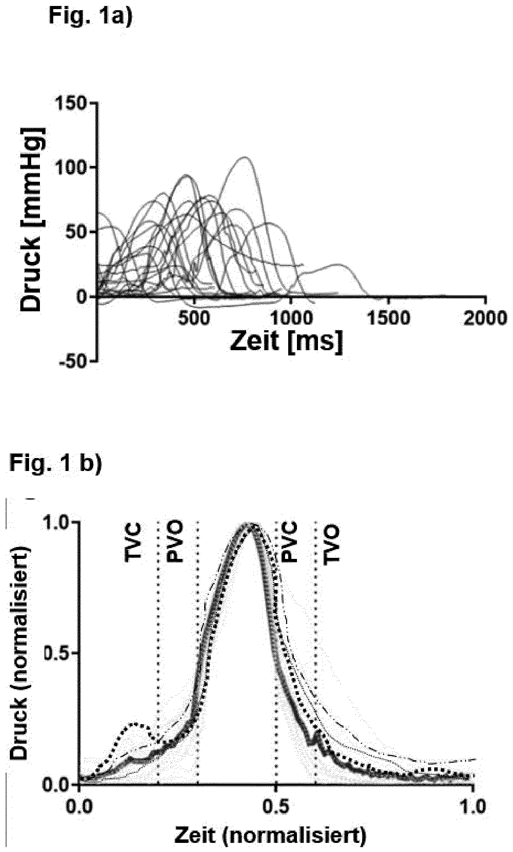

Das erfindungsgemäße Verfahren umfasst dabei die folgenden Schritte.
Ia) Bestimmung des zeitlichen Verlaufs des Blutdrucks während der isovolumetrischen Kontraktion (IVK) und der isovolumetrischen Relaxation (IVR) für wenigstens eine Herzkammer über wenigstens eine Periode eines Herzschlages einer Person. Zunächst erfolgt die Bestimmung des zeitlichen Verlaufs des Blutdrucks während der IVK und der IVR für wenigstens eine Herzkammer. Pro Messung des Druckverlaufs über eine Periode eines Herzschlages wird eine Druckkurve generiert. Die Bestimmung erfolgt dabei vorzugsweise mittels Herzsonographie. Ein beispielhaftes Ensemble solcher Druckkurven zeigt die Abbildung Fig.1a.
Ib) Bestimmung der Geschwindigkeit (v) des Regurgitationsflusses über der Trikuspidalklappe aus mindestens einer Druckkurve der isovolumetrischen Kontraktion (IVK) und isovolumetrischen Relaxation (IVR) bestimmt. aus Schritt Ia) Die Bestimmung des Regurgitationsflusses erfolgt dabei über eine Doppler-Flussmengen-Messung.
Ic) Berechnung des maximalen rechtsventrikulären Drucks (Pmax) aus der Geschwindigkeit (v) des Regurgitationsflusses aus Schritt Ib). Diese Berechnung ist ein Routine-Verfahren der Herzultraschallmessung. Dabei dient die Bernoullie Gleichung zur Abschätzung des Druckes aus der Geschwindigkeit. Es gilt dabei Pmax~(4v)². Der maximale rechtsventrikuläre Druck Pmax dient in den folgenden Schritten zur individuellen Kalibrierung der Druck/Zeit-Kurve bezüglich des Druckes.
Id) Kalibrieren der mindestens einen Druckkurve aus Schritt Ia) mittels der IVR -und IVK-Zeiten aus Schritt Ia) sowie des maximalen rechtsventrikuläre Druckes (Pmax) aus Schritt Ic) der zu untersuchenden Person umso eine kalibrierte Druckkurve für diese Person zu erhalten. Bei der Kalibrierung wird die gemessene wenigstens eine Druckkurve mit Reverenzmessungen erhaltenen Druckkurven verglichen und so angepasst, dass die Abweichungen der mittleren Kurvenform von einer Reverenzkurvenform über eine Periode eines Herzschlages minimal ist. Bei den Referenzmessungen handelt es sich vorzugsweise um Messungen mittels Routine-Rechtsherzkatheter.
Ie) Normalisierung mindestens einer Druckkurve aus Schritt Id) der zu untersuchenden Herzkammer umso eine kalibrierte normalisierte Druckkurve zu erhalten. Für die Normalisierung in Schritt Ie) werden die IVR -und IVK-Zeiten aus Schritt Ia) sowie wenigstens eine während des Verfahrens gewonnenen Druckkurve verwendet. Normalisierung bedeutet hier die Skalierung des Wertebereichs des Blutdrucks und der Zeit auf einen bestimmten Bereich, zwischen 0 und 1. Die Normalisierung dient dazu die Ergebnisse mit unterschiedlicher Grundlage vergleichbar zu machen. Die Normalisierung wenigstens einer Druckkurve erfolgt mittels der IVR -und IVK-Zeiten aus Schritt Ia) sowie des maximalen rechtsventrikuläre Druckes Pmax aus Schritt Ic) der Person umso eine normalisierte Druckkurve der jeweiligen Person zu erhalten. Ein beispielhaftes Ensemble solcher kalibrierter Druckkurven zeigt die Abbildung Fig.1b.
   Die Normalisierung der wenigstens einen Druckkurve erfolgt durch Zusammenfassen der Messungen über jeweils eine Periode eines Herzschlages und erfolgt unter Verwendung des Zeitablaufs der Herzkontraktion in den folgenden drei Schritte:
   Ieₐ) zunächst erfolgt eine Zuordnung der Zeitpunkte der Eröffnung und Schließung der Mitral- und Aortenklappe zur jeweiligen Druckspur, an Hand der Messdaten aus Schritt la.
   Ie_{b}) Danach wird die wenigstens eine Druckkurve entlang der Zeitachse zwischen den Zeitpunkten aus Schritt Ieₐ) gedehnt oder gestaucht, sodass die Ventilereignisse für alle Druckkurven zusammenfallen.
   Ie_{c}) Anschließend werden die wenigstens eine zeitlich gedehnten oder gestauchten Druckkurven aus Schritt Ie_{b}) vertikal skaliert, um den gleichen maximalen rechtsventrikulären Druck Pmax zu erhalten. Anschließend wird eine gemittelte Wellenform berechnet.
Der maximale rechtsventrikuläre Druck Pmax wird zur Skalierung der Amplitude der Druckkurve verwendet.

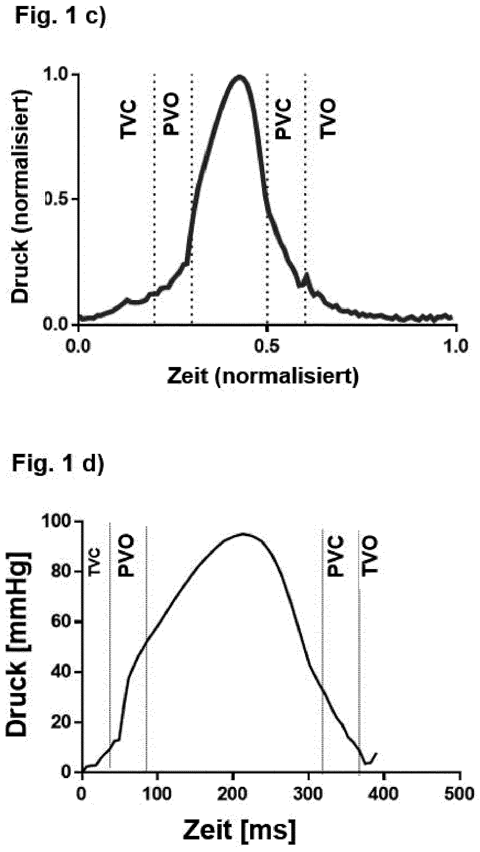

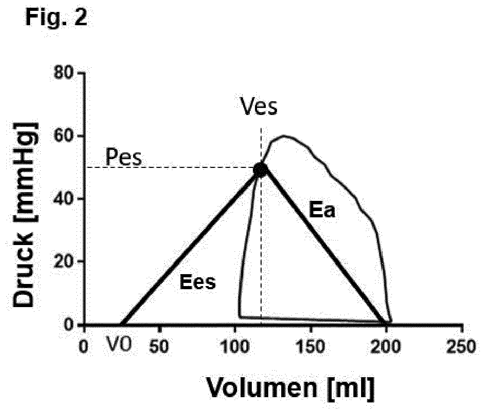

Optional erfolgt nach der Kalibrierung noch eine Mittelung über alle kalibrierten normalisierten Druckkurven. Dies ist beispielhaft in Abbildung Fig.1c gezeigt.
II. Ermittlung des endsystolischen Druckes (Pes) aus der wenigstens einen kalibrierten normalisierten Druckkurve aus Schritt Ie). Dies ist beispielhaft in Abbildung Fig.1d gezeigt.
III. Bestimmung des zeitlichen Verlaufs des Blutvolumens während der isovolumetrischen Kontraktion (IVK) und der isovolumetrischen Relaxation (IVR) für wenigstens eine Herzkammer über wenigstens eine Periode eines Herzschlages einer Person, wobei pro Messung des Volumenverlaufs über eine Periode eines Herzschlages eine Volumenkurve generiert wird. Die Bestimmung erfolgt dabei vorzugsweise mittels Herzsonographie. Dies ist beispielhaft in Abbildung Fig.2 gezeigt.
IV. Ermittlung des endsystolischen Volumens Ves aus wenigstens einer Volumenkurve aus Schritt III.
V. Ermittlung des Intercept V0 aus dem endsystolischen Volumen Ves aus Schritt IV. Dabei gilt in guter Näherung V0 = -50,01ml+0,7*Ves. Diese Formel wurde empirisch aus direkten Messungen von V0 bestimmt. Dabei wurde die Goldstandardmethode angewendet, die über die sog. Multi-Beat-Methode eine Vorlastreduktion erwirkt, welche durch kurzzeitigen Verschluss der Vena cava inferior (untere Hohlvene) erreicht wurde. Die Steigung des endsystolischen Drucks je Volumen ist die Kontraktilität (endsystolische Elastance) und schneidet die x-Achse (Volumen) am Punkt V0. V0 hat eine lineare Beziehung zum endsystolischen Volumen. Aus dieser linearen Beziehung ergibt sich die oben genannte Formel für V0. Der Intercept V0 ist dabei der X-Achsenabschnitt der ventrikulären endsystolischen Druck-Volumen-Beziehungslinie. Dies ist beispielhaft in der Abbildung Fig. 2 gezeigt.
VI. Ermittlung der lastunabhängigen Kontraktilität Ees der zu untersuchenden Herzkammer als Steigung aus endsystolischem Druck Pes aus Schitt II und dem Intercept V0 aus Schittt V. Die lastunabhängige Kontraktilität ist dabei Steigung der Volumenkurve zwischen dem Intercept V0 und dem endsystolischem Druck Pes und es gilt somit Ees= Pes / (Ves-V0).

In einer Weiterbildung der Erfindung ist es möglich das erfindungsgemäße Verfahren mit einer Ultraschallüberwachungsvorrichtung 1 durchzuführen.

Diese Vorrichtung umfasst mehrere Elemente:

### Ultraschallmesselement

Das Ultraschallmesselement (z.B. eine Ultraschallsonde), ist an einer Körperoberfläche eines zu untersuchenden Patienten anbringbar und so ausgebildet, dass es die Körperoberfläche abtasten und ein Echosignal erfassen kann.

Alternativ, aber nicht unter die Erfindung fallend, ist das Ultraschallmesselement in den Patienten einbringbar und so ausgebildet dass ein Echosignal im Körperinneren erfasst werden kann. Dies ist z.B. bei der transösophagealen Echokardiografie (TEE) der Fall. Hier erfolgt die Ultraschalluntersuchung des Herzens von der Speiseröhre aus. Für den Patienten läuft die Untersuchung ähnlich ab wie eine Magenspiegelung.

In einer Weiterbildung der Ultraschallüberwachungsvorrichtung 1, aber nicht unter die Erfindung fallend, umfasst diese mehrere Ultraschallsonden, die an dem Ventrikel, der Aorta und den peripheren Arterien des Patienten angebracht werden können, um ein synchronisiertes Echtzeit-Scannen durchzuführen.

### Blutdruckmesselement

Das Blutdruckmesselement ist dabei so ausgebildet, dass es wenigstens einen Blutdruckparameter eines zu untersuchenden Patienten messen kann. Blutdruckparameter können dabei vorzugsweise sein: Blutdruck, periphere arterielle Pulswelleninformationen, einen systolischen Druck, einen diastolischen Druck und mittleren arteriellen Druck. Das Blutdruckmesselement ist dabei vorzugsweise ein Aneroid-Blutdruckmessgerät oder ein Finger-Blutdruckmessgerät.

### Verarbeitungselement

Das Verarbeitungselement ist dabei so ausgebildet, dass es ein Echosignal des Ultraschallmesselements empfangen und aus dem Echosignal wenigstens einen Blutflussparameter berechnen kann. Weiterhin ist es so ausgebildet, dass es wenigstens einen myokardmechanischen Parameter (z.B. lastunabhängige Kontraktilität) aus wenigstens einem Blutflussparameter und wenigstens einem Blutdruckparameter berechnen kann.

Myokardmechanische Parameter können dabei vorzugsweise sein: der X-Achsenabschnitt der ventrikulären endsystolischen Druck-Volumen-Beziehungslinie Vo und die lastunabhängige Kontraktilität Ees.

Blutflussparameter können dabei vorzugsweise sein: Aorten-Vorwärts-Doppler-Blutflusssignale, 2D- und peripheres arterielles Doppler-Flusssignal zur Bestimmung des Regurgitationsflusses.

Weiterhin ist das Verarbeitungselement so ausgebildet, dass es den Blutdruck, wenigstens einen Blutflussparameter und wenigstens einen myokardmechanischen Parameter anzeigen und an ein Anzeigeelement weiterleiten kann.

In einer Weiterbildung der Ultraschallüberwachungsvorrichtung 1 ist das Verarbeitungselement so ausgebildet, dass es das Echosignal digital zu einem digitalen Verarbeitungssignal verarbeiten kann, um eine numerische Parameter, Wellenform- oder Trendgraphenberechnung gemäß dem digitalen Verarbeitungssignal zu erhalten, wobei das digitale Verarbeitungssignal ausgewählt wird aus der Gruppe bestehend aus: HF-Signal, Basisbandsignal und Hüllkurvensignal.

### Anzeigeelement

Das Anzeigeelement ist dabei so ausgebildet, dass es den Blutdruck, wenigstens einen Blutflussparameter und wenigstens einen myokardmechanischen Parameter des Verarbeitungselementes empfangen und anzeigen kann.

Weiterhin ist es möglich, dass Ausführungsformen der Erfindung in Hardware, Firmware, Software oder einer beliebigen Kombination davon implementiert sein können.

Weiterbildungen der Erfindung können auch als Anweisungen implementiert werden, die auf einem computerlesbaren Speichermedium gespeichert sind, das von einem oder mehreren Prozessoren gelesen und ausgeführt werden kann.

Das computerlesbare Speichermedium kann Magnetplattenspeichermedien, optische Speichermedien, Nur-Lese-Speicher (ROM), Direktzugriffsspeicher (RAM) und dergleichen umfassen.

Dabei gilt, dass, wenn ein Element als "verbunden" oder "gekoppelt" mit einem anderen Element bezeichnet wird, es direkt mit dem anderen Element verbunden oder gekoppelt sein kann oder dass dazwischenliegende andere Elemente vorhanden sein können.

### Ausführungsbeispiele

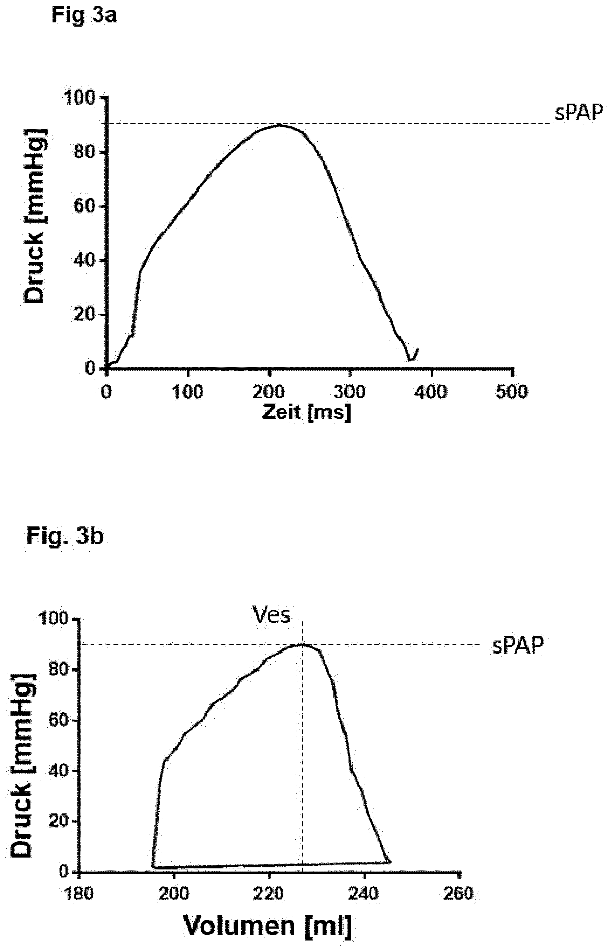

Beispielhafte Messdaten eines Patienten zeigt die Abbildung Fig.3. Die Abbildung Fig.3a zeigt dabei eine gemittelte kalibrierte normalisierte Druckkurve, wie sie nach Schritt I vorliegt. Die Messung fand hier am rechten Ventrikel statt.

Die Abbildung Fig.3b die Druckvolumenkurve des Patenten. Die Druckvolumenkurve kombiniert den Verlauf der Bestimmung des zeitlichen Verlaufs des Blutdrucks aus Schritt I und des Blutvolumens aus Schritt III.

Die Echokardiografiedaten ergeben folgende Werte:

| Beginn der IVK | Ende der IVK | Beginn der IVR | End der IVR | sPAP |
|---|---|---|---|---|
| 0s | 40s | 327s | 384s | 90 mmHg |

Der pulmonalarterielle Druck sPAP entspricht dem endsystolischem Druck Pes und wurde, wie in Schritt II beschrieben, aus der gemittelten kalibrierten normalisierten Druckkurve ermittelt.

Die Ermittlung des endsystolische Volumen Ves in Schritt IV ergab einen Wert von 225ml. Dieser Wert wurde der Volumenkurve der Abbildung Fig.3b entnommen.

Anschließend wurde Schritt V durchgeführt um den Intercept V0 zu bestimmen.
Dabei gilt V0 = -50,01+0,7*Ves
Damit ist der Intercept V0= 107,49ml
Damit wurde Schritt VI durchgeführt um die lastunabhängige Kontraktilität Ees zu bestimmen.
Dabei gilt für die lastunabhängige Kontraktilität Ees= Pes / (Ves-V0)
Ees= 90 mmHg/(225-107,49)ml
Damit beträgt lastunabhängige Kontraktilität 0,77 mmHg/ml.

### Abbildungslegenden und Bezugszeichenliste

Fig. 1 zeigt in der Teilabbildung 1a) Messwerte einer beispielhaften Messung für den zeitlichen Verlauf des Blutdrucks über eine Schlagperiodendauer. Teilabbildung 1b zeigt die normalisierten Verläufe des Blutdrucks. Die Teilabbildung 1c) zeigt den gemittelten Verlauf des Blutdrucks. In der Teilabbildung 1 d) zeigt die Werte für den Blutdruck und Zeit nach der Umrechnung aus normalisierten Einheiten in reguläre Einheiten.
Fig.2 zeigt beispielhaft das endsystolischen Volumen Ves in Abhängigkeit vom endsystolischen Druck Pes. Das Volumen wurde hier mittels Ultraschallmessungen bestimmt.
Fig.3. zeigt die Messwerte eines Beispielpatienten

### Bezugszeichen:

TVC: Tricuspidal Valve closure; Schluss der Trikuspidalklappe,
TVO: Tricuspidal Valve Opening, Öffnung der Trikuspidalklappe,
PVC: Pulmonary Valve Closure, Schluss der Pulmonalklappe,
PVO: Pulmonary Valve Opening: Öffnung der Pulmonalklappe

## Patentansprüche

1. Verfahren zur nichtinvasiven Bestimmung der lastunabhängigen Kontraktilität (Ees) wenigstens einer zu untersuchenden Herzkammer einer Person umfassend folgende Schritte:
la) Bestimmung des zeitlichen Verlaufs des Blutdrucks während der isovolumetrischen Kontraktion (IVK) und der isovolumetrischen Relaxation (IVR) für wenigstens eine Herzkammer über wenigstens eine Periode eines Herzschlages einer Person, wobei pro Messung des Druckverlaufs über eine Periode eines Herzschlages eine Druckkurve generiert wird,
lb) Bestimmung der Geschwindigkeit (v) des Regurgitationsflusses über der Trikuspidalklappe aus mindestens einer Druckkurve aus Schritt Ia) und einer Bestimmung des Regurgitationsflusses über eine Doppler-Flussmengen-Messung,
Ic) Berechnung des maximalen rechtsventrikulären Drucks (Pmax) aus der Geschwindigkeit (v) des Regurgitationsflusses aus Schritt Ib),
Id) Kalibrieren mindestens einer Druckkurve aus Schritt Ia) mittels der IVR -und IVK-Zeiten aus Schritt Ia) sowie des maximalen rechtsventrikuläre Druckes (Pmax) der zu untersuchenden Person, um so eine kalibrierte Druckkurve für diese Person zu erhalten.
Ie) Normalisierung mindestens einer Druckkurve aus Schritt Id) der zu untersuchenden Herzkammer, um so eine kalibrierte normalisierte Druckkurve zu erhalten,
II. Ermittlung des endsystolischen Druckes (Pes) aus der kalibrierten normalisierten Druckkurve aus Schritt Ie),
III. Bestimmung des zeitlichen Verlaufs des Blutvolumens während der isovolumetrischen Kontraktion (IVK) und der isovolumetrischen Relaxation (IVR) für wenigstens eine Herzkammer über wenigstens eine Periode eines Herzschlages einer Person, wobei pro Messung des Volumenverlaufs über eine Periode eines Herzschlages eine Volumenkurve generiert wird,
IV. Ermittlung des endsystolischen Volumens (Ves) aus wenigstens einer der Volumenkurve aus Schritt III,
V. Ermittlung des Intercept (V0) aus dem endsystolischen Volumen (Ves) aus Schritt IV,
VI. Ermittlung der lastunabhängigen Kontraktilität (Ees) der zu untersuchenden Herzkammer einer Person als Steigung aus endsystolischem Druck (Pes) aus Schritt II, dem endsystolischen Volumens (Ves) aus Schritt IV und dem Intercept (V0) aus Schittt V, wobei die lastunabhängige Kontraktilität die Steigung der Volumenkurve zwischen dem Intercept (V0) und dem endsystolischem Druck (Pes) ist und somit Ees= Pes / (Ves-V0) gilt.

## Claims

1. Method for the non-invasive determination of the load-independent contractility (Ees) of at least one cardiac ventricle of a person to be examined, comprising the following steps:
la) determining the time course of the blood pressure during isovolumetric contraction (IVC) and isovolumetric relaxation (IVR) for at least one heart ventricle over at least one period of a heartbeat of a person, wherein a pressure curve is generated by measurement of the pressure course over one period of a heartbeat.
Ib) Determining the velocity (v) of the regurgitant flow across the tricuspid valve by Doppler-flow-rate measurement from at least one pressure curve from step Ia).
Ic) calculating the maximum right ventricular pressure (Pmax) from the velocity (v) of regurgitant flow from step Ib)
ld) calibrating at least one pressure curve from step Ia) using the IVR and IVK times from step Ia) and the maximum right ventricular pressure (Pmax) of the subject under examination to obtain a calibrated pressure curve for this subject.
Ie) Normalisation of at least one pressure curve from step Id) of the ventricle to be examined in order to obtain a calibrated normalised pressure curve.
II. determination of the end-systolic pressure (Pes) from the calibrated normalised pressure curve from step Ie).
III. determining the time course of the blood volume during isovolumetric contraction (IVC) and isovolumetric relaxation (IVR) for at least one heart ventricle over at least one period of a heartbeat of a person, wherein a volume curve is generated per measurement of the volume course over one period of a heartbeat.
IV. Determining the end-systolic volume (Ves) from at least one of the volume curves from step III.
V. Determine the intercept (V0) from the end-systolic volume (Ves) from step IV.
VI. determining the load-independent contractility (Ees) of the cardiac ventricle of a person to be examined as the slope of the end-systolic pressure (Pes) from step II, the end-systolic volume (Ves) from step IV and the intercept (V0) from step V, wherein the load-independent contractility is the slope of the volume curve between the intercept (V0) and the end-systolic pressure (Pes) and thus Ees= Pes / (Ves-V0) applies.

## Revendications

1. Méthode de détermination non invasive de la contractilité indépendante de la charge (Ees) d'au moins un ventricule cardiaque d'une personne à examiner, comprenant les étapes suivantes:
la) Détermination de l'évolution temporelle de la pression sanguine pendant la contraction isovolumétrique (IVC) et la relaxation isovolumétrique (IVR) pour au moins un ventricule cardiaque sur au moins une période d'un battement de coeur d'une personne, une courbe de pression étant générée par la mesure de l'évolution de la pression sur une période d'un battement de coeur.
Ib) Détermination de la vitesse (v) du flux régurgitant à travers la valve tricuspide par mesure du débit Doppler à partir d'au moins une courbe de pression obtenue à l'étape Ia).
Ic) calcul de la pression ventriculaire droite maximale (Pmax) à partir de la vitesse (v) du flux régurgitant obtenue à l'étape Ib)
Id) étalonner au moins une courbe de pression de l'étape Ia) en utilisant les temps IVR et IVK de l'étape Ia) et la pression ventriculaire droite maximale (Pmax) du sujet examiné pour obtenir une courbe de pression étalonnée pour ce sujet.
Ie) Normalisation d'au moins une courbe de pression de l'étape Id) du ventricule à examiner afin d'obtenir une courbe de pression normalisée calibrée.
II. Détermination de la pression endosystolique (Pes) à partir de la courbe de pression normalisée calibrée de l'étape Ie).
III. déterminer l'évolution temporelle du volume sanguin pendant la contraction isovolumétrique (IVC) et la relaxation isovolumétrique (IVR) pour au moins un ventricule cardiaque sur au moins une période d'un battement de coeur d'une personne, une courbe de volume étant générée par mesure de l'évolution du volume sur une période d'un battement de coeur.
IV. Détermination du volume endosystolique (Ves) à partir d'au moins une des courbes de volume de l'étape III.
V. Déterminer l'ordonnée à l'origine (V0) à partir du volume de fin de systole (Ves) de l'étape IV.
VI. déterminer la contractilité indépendante de la charge (Ees) du ventricule cardiaque d'une personne à examiner comme étant la pente de la pression endo-systolique (Pes) de l'étape II, le volume endo-systolique (Ves) de l'étape IV et l'ordonnée à l'origine (V0) de l'étape V, la contractilité indépendante de la charge étant la pente de la courbe de volume entre l'ordonnée à l'origine (V0) et la pression endo-systolique (Pes) et donc Ees= Pes / (Ves-V0) s'applique.
